(19) Europäisches Patentamt
European Patent Office
Office européen des brevets

(11) Veröffentlichungsnummer: **0 244 727 B1**

(12) **EUROPÄISCHE PATENTSCHRIFT**

(45) Veröffentlichungstag der Patentschrift: **01.08.90**

(51) Int. Cl.⁵: **C 07 C 265/12,** C 08 G 18/77

(21) Anmeldenummer: **87106082.8**

(22) Anmeldetag: **27.04.87**

(54) **1,4-Bis-(4-isocyanatophenoxy)- benzole, ein Verfahren zu ihrer Herstellung und ihre Verwendung bei der Herstellung von Polyurethankunststoffen.**

(30) Priorität: **09.05.86 DE 3615723**

(43) Veröffentlichungstag der Anmeldung:
**11.11.87 Patentblatt 87/46**

(45) Bekanntmachung des Hinweises auf die Patenterteilung:
**01.08.90 Patentblatt 90/31**

(84) Benannte Vertragsstaaten:
**BE DE FR GB IT**

(56) Entgegenhaltungen:
**EP-A-0 183 115**

**Chemical Abstracts, Registry Handbook, 1965-1971**

(73) Patentinhaber: **BAYER AG**
**D-5090 Leverkusen 1 Bayerwerk (DE)**

(72) Erfinder: **Heitkämper, Peter, Dr.**
**Fliederweg 14**
**D-4047 Dormagen 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

**Beschreibung**

Die Erfindung betrifft 1,4-Bis-(4-isocyanatophenoxy)-benzole, ein Verfahren zu ihrer Herstellung durch Umsetzung von 1,4-Bis-(4-aminophenoxy)-benzolen oder ihrer Addukte mit Chlorwasserstoff oder Kohlendioxid mit Phosgen, sowie ihre Verwendung als Aufbaukomponente bei der Herstellung von Polyurethankunststoffen.

Die Eigenschaften von Polyurethankunststoffen, insbesondere von Polyurethanelastomeren hängen u.a. wesentlich von der Natur des bei der Herstellung der Kunststoffe eingesetzten Polyisocyanats ab. Besonders hochwertige Polyurethanelastomere werden so beispielsweise bei Verwendung von 1,5-Diisocyanatonaphthalin als Diisocyanatkomponente erhalten. Insbesondere zeichnen sich Gießelastomere auf Basis dieses Diisocyanats durch hervorragende mechanische Eigenschaften aus (vgl. z.B. Becker, Braun, Kunststoff-Handbuch, Band 7, 2. Auflage (1983), Carl-Hanser-Verlag), 1,5-Diisocyanatonaphthalin ist jedoch mit dem Nachteil behaftet, daß der zu seiner Herstellung eingesetzte Grundrohstoff, Naphthalin, nur in beschränkten Mengen zur Verfügung steht. Die Nitrierung von Naphthalin führt außerdem zwangsläufig zu einem Isomerengemisch von Nitronaphthalinen, aus dem 1,5-Dinitronaphthalin isoliert werden muß. Die destillative Reinigung des 1,5-Diisocyanatonaphthalins, welches aus der Dinitroverbindung durch Hydrierung und anschließende Phosgenierung des resultierenden Diamins erhalten wird, bereitet ebenfalls Schwierigkeiten, da es zur Sublimation neigt. Dies alles führt dazu, daß 1,5-Diisocyanatonaphthalin mit hohen Kosten belastet ist.

Auch die Verarbeitung von 1,5-Diisocyanatonaphthalin ist häufig problematisch, weil sein Schmelzpunkt und sein Dampfdruck relativ hoch sind. Diese Eigenschaften erlauben es oft nicht, 1,5-Diisocyanatonaphthalin ohne weiteres als Schmelze umzusetzen. Es sind dann technisch aufwendige Verarbeitungsmethoden und Schutzmaßnahmen erforderlich, um chemische und arbeitsmedizinische Schwierigkeiten zu vermeiden.

Es hat daher nicht an Versuchen gefehlt, für 1,5-Diisocyanatonaphthalin als Diisocyanatkomponente bei der Herstellung hochwertiger Polyurethankunststoff einen gleichwertigen Ersatz zu finden.

So ist beispielsweise in DE—OS 31 38 421 und DE—OS 31 38 422 die Herstellung von Polyurethan-Elastomeren unter Verwendung von 4,4'-Diisocyanato-diphenylethan-(1,2) als Diisocyanat-Komponente beschrieben. Zwar können mit diesem Diisocyanat Kunststoffe mit guten mechanischen Eigenschaften erhalten werden; jedoch ist die Herstellung von 4,4'-Diisocyanato-diphenylethan-(1,2) sehr umständlich und aufwendig und bis jetzt auch technisch schwer realisierbar.

Weiterhin sind zahlreiche Versuche unternommen worden, das vergleichsweise kostengünstige 4,4'-Diisocyanatodiphenylmethan anstelle von 1,5-Diisocyanatonaphthalin zur Herstellung von hochwertigen Polyurethanelastomeren zu verwerden, jedoch sind bislang alle Versuche gescheitert, auf Basis dieses Diisocyanats Polyurethanelastomere herzustellen, die bezüglich ihrer mechanischen und thermischen Eigenschaften den Polyurethanelastomeren auf Basis von 1,5-Diisocyanatonaphthalin entsprechen.

Es war daher die Aufgabe der vorliegenden Erfindung, neue Diisocyanate zur Verfügung zu stellen, welche bezüglich ihrer Eignung zur Herstellung von hochwertigen Polyurethanelstomeren dem 1,5-Diisocyanatonaphthalin vergleichbar sind, und welche sich nach einfachen Verfahren kostengünstig herstellen lassen.

Die eigenen älteren Vorschläge (europäische Patentanmeldung 85 114 308.1 entsprechend US-Anmelding 796 128 vom 08.11.1985 bzw. europäische Patentanmeldung 86 102 100.4 entsprechend US-Anmeldung 829 041 vom 13.02.1986) stellen erste Versuche zur Lösung dieser Aufgabe dar. Jetzt wurde in Ergänzung hierzu gefunden, daß die gestellte Aufgabe auch durch die Bereitstellung von 1,4-Bis-(4-isocyanatophenoxy)-benzolen bzw, dem Verfahren zu ihrer Herstellung gelöst werden kann.

Gegenstand der Erfindung sind 1,4-Bis-(4-isocyanatophenoxy)-benzole der allgemeinen Formel

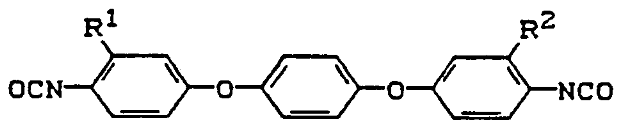

in der

$R^1$ und $R^2$ gleich oder verschieden sind und die Bedeutung eines Wasserstoffatoms oder einer Methylgruppe haben.

Gegenstand der Erfindung ist auch ein Verfahren zur Herstellung von 1,4-Bis-(4-isocyanatophenoxy)-benzolen der genannten Formel, dadurch gekennzeichnet, daß man 1,4-Bis-(4-aminophenoxy)-benzole der allgemeinen Formel

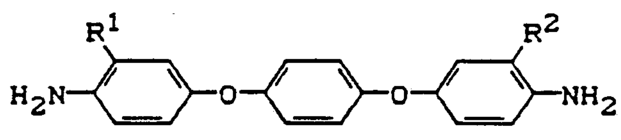

in der $R^1$ und $R^2$ die genannte Bedeutung haben, oder deren Addukte mit Chlorwaserstoff oder Kohlendioxid in an sich bekannter Weise mit Phosgen umsetzt.

Gegenstand der Erfindung ist auch die Verwendung von 1,4-Bis-(4-isocyanatophenoxy)-benzolen der genannten Formel als Aufbaukomponente bei der Herstellung von Polyurethankunststoffen nach dem Isocyanat-Polyadditionsverfahren.

Die bei der Durchführung des erfindungsgemäßen Verfahrens als Ausgangsmaterial einzusetzenden Diamine sind leicht zugänglich. Sie können beispielsweise aus den entsprechenden Dinitroverbindungen durch Reduktion mit unedlen Metallen, z.B. Zink oder Eisen, in Gegenwart von Säuren oder durch katalytische Hydrierung hergestellt werden.

Die den Diaminen zugrunde liegenden Dinitroverbindungen sind ebenfalls leicht zugänglich. Sie können beispielsweise durch Kondensation der Hydrochinondialkalisalze mit 4-Nitrochlorbenzol und/oder mit 4-Nitro-3-methyl-chlor-benzol hergestellt werden (vgl. Chemical Abstracts *54*, 353 f (1960)).

Selbstverständlich können für das erfindungsgemäße Verfahren die einzusetzenden Diamine und die ihnen zugrunde liegenden Dinitroverbindungen auch nach jeder beliebigen anderen Methode hergestellt werden.

Die beim erfindungsgemäßen Verfahren einzusetzenden Diamine sind: 1,4-Bis-(4-aminophenoxy)-benzol, 1-(4-Amino-3-methyl-phenoxy)-4-(4-amino-phenoxy)-benzol und 1,4-Bis-(4-amino-3-methyl-phenoxy)-benzol. Beliebige Gemische dieser drei Diamine können ebenfalls verwendet werden.

Die erfindungsgemäß zu phosgenierenden Diamine können beim erfindungsgemäßen Verfahren in technischer Reinheit, wie sie bei ihrer Herstellung anfallen, oder auch in gereinigter Form zum Einsatz gelangen. Die Reinigung kann beispielsweise durch Umkristallisieren aus Chlorbenzol oder destillativ erfolgen.

Beim erfindungsgemäßen Verfahren können die Diamine als solche oder auch in Form ihrer Additionsverbindungen mit Chlorwasserstoff oder mit Kohlendioxid zum Einsatz gelangen. Die erfindungsgemäße Phosgenierung erfolgt im übringen nach an sich bekannten Methoden, wie sie beispielsweise in Liebigs Annalen der Chemie, Band 562, Jahrgang 1949, Seiten 75 bis 109, in Ullmanns Enzyklopädie der technischen Chemie Band 14, 4. Auflage, 1977, Seiten 350 bis 354 oder in Houben-Weyl, Methoden der organischen Chemie, Band E4, 4. Auflage, 1963, Seiten 741 bis 753 beschrieben sind.

Die Umsetzung kann kontinuierlich oder diskontinuierlich bevorzugt in Gegenwart eines inerten Lösungsmittels durchgeführt werden. Geeignete Lösungsmittel sind die für Phosgenierungen üblicherweise verwendeten Lösungsmittel, wie aliphatische, cycloaliphatische oder aromatische Kohlenwasserstoffe, Halogenkohlenwasserstoffe, Nitrokohlenwasserstoffe, aliphatisch-aromatische Ether, aromatische Ether, Carbonsäureester, Carbonsäurenitrile, Sulfone, Phosphorsäurehalogenide oder Phosphorsäureester. Als Beispiele für geeignete Lösungsmitel seien genannt: Trimethylpentan, Decahydronaphthalin, Toluol, 1,2-Dichlorethan, Chlorbenzol, Chlortoluol, 1,2-Dichlorbenzol, Nitrobenzol, Anisol, Phenetol, Diphenylether, Essigsäurebutylester, Tetramethylsulfon, Phosphoroxychlorid, Phosphorsäuretrimethylester. Bevorzugt wird technisches Chlorbenzol oder technisches 1,2-Dichlorbenzol als Lösungsmittel verwendet. Beliebige Gemische der beispielhaft genannten Lösungsmittel können selbstverständlich ebenfalls eingesetzt werden. In einigen der beispielhaft genannten Lösungsmittel weisen die 1,4-Bis-(4-aminophenoxy)-benzole bei niedrigen Temperaturen nur eine geringe Löslichkeit auf.

Bei der Durchführung des erfindungsgemäßen Verfahrens erfüllen demzufolge die beispielhaft genannten Lösungsmittel insbesondere bei den niedrigen Phosgeniertemperaturen vor allem die Funktion eines Suspendiermittels für das Diamin bzw. dessen Chlorwasserstoff- oder Kohlendioxid-Addukte und erste bei höheren Temperaturen und mit zunehmender Überführung des Ausgangsmaterials in das Diisocyanat die Funktion eines echten Lösungsmittels für das Ausgangsmaterial und insbesondere das Verfahrensprodukt.

Bei den beim erfindungsgemäßen Verfahren einzusetzenden Gemischen aus zu phosgenierendem Ausgangsmaterial und Lösungsmittel handelt es sich im allgemeinen um "Lösungssuspensionen" mit einem Gehalt an Diamin bzw. Diaminaddukt der oben beispielhaft genannten Art von ca. 2 bis 70 Gew.-%. Der Begriff "Lösungssuspension" soll andeuten, daß die Ausgangsmaterialien, insbesondere im Falle der bevorzugten Verwendung der Diamine, teilweise gelöst und teilweise suspendiert vorliegen.

Die erfindungsgemäße Phosgenierungsreaktion kann beispielsweise nach dem bekannten Prinzip der "Kalt-Heiß-Phosgenierung" zweistufig oder nach dem Prinzip der "Heiß-Phosgenierung" einstufig erfolgen. Bei der "Kalt-Heiß-Phosgenierung" erfolgt die Umsetzung des zu phosgenierenden Ausgangsmaterials zu Beginn der Reaktion im allgemeinen bei −20°C bis +40°C, vorzugsweise −10°C bis +30°C, und die anschließende Heißphosgenierung bei 40°C bis 260°C, vorzugsweise 80°C bis 220°C. Bei dieser "Kalt-Heiß-Phosgenierung" kann der Bereich zwischen der Anfangstemperatur und der erhöhten Temperatur gleichmäßfig oder in Sprüngen durchlaufen werden.

Bei der "Heißphosgenierung" kommt das zu phosgenierende Ausgangsmateral mit dem Phosgen sofort bei Temperaturen von 40°C bis 260°C, vorzugsweise 80°C bis 220°C, in Kontakt.

Die genannte "Kalt-Heiß-Phosgenierung" ist die bevorzugte Verfahrensweise im Falle der Phosgenierung des Diamins (anstelle der Chlorwasserstoff- bzw. Kohlendioxid-Addukte). Hierbei erfolgt bei den genannten tiefen Temperaturen keine nennenswerte Umsetzung zwischen dem suspendierten Diamin und dem zugefügten Phosgen. Erst bei der nachfolgenden Erhöhung der Temperatur beginnt das Diamin mit dem Phosgen entsprechend seiner zunehmenden Löslichkeit zu reagieren.

Bei allen Varianten der erfindungsgemäßen Phosgenierung erfolgt diese vorzugsweise unter Normaldruck oder bei erhöhtem Druck. Der Reaktionsdruck liegt im allgemeinen bei 0,9 bis 100, vorzugsweise 1 bis 60 bar.

Im allgemeinen wird bei der erfindungsgemäßen Phosgenierungsreaktion das zu phosgenierende Ausgangsmaterial mit einer 1- bis 10-fachen, vorzugsweise 1,05- bis 6-fachen stöchiometrischen Menge an Phosgen zusammengebracht. Die genannte Phosgenmenge kann in einer Portion oder auch in Teilmengen in das Reaktionsgemisch eingegeben werden. Es kann vorteilhaft sein, z.B. bei einer diskontinuierlichen Arbeitsweise, erst einen Teil der zu verwendenden Phosgenmenge in das Reaktionsgemisch einzubringen und den restlichen Anteil in weiteren Portionen oder stetig über einen längeren Zeitraum verteilt in das Reaktionsgemisch einzubringen.

Grundsätzlich läßt sich die erfindungsgemäße Phosgenierung der Diamine durch Zugabe von Katalysatoren, beispielsweise Dimethylformamid, und/oder Säureakzeptoren, beispielsweise Pyridin, beschleunigen. Im allgemeinen jedoch sind die Reaktionsgeschwindigkeiten bei der Phosgenierung des Diamins auch ohne Zugabe eines derartigen Katalysators ausreichend.

Die Reaktionsdauer bei der erfindungsgemäßen Phosgenierung ist von den angewandten Reaktionsbedingungen, insbesondere von den Reaktionstemperaturen, vom Phosgen-Überschuß, von der Verdünnung mit Lösungsmittel und von gegebenenfalls zugegebenen Katalysatoren und/oder Säureakzeptoren abhängig.

Nach Beendigung der Phosgenierungsreaktion wird das Reaktionsgemisch in an sich bekannter Weise durch Abtrennung von gasförmigen Bestandteilen (Chlorwasserstoff, überschüssiges Phosgen) und destillativer Entfernung des Lösungsmittels aufgearbeitet. Vor der destillativen Entfernung des Lösungsmittels können gegebenenfalls vorliegende feste Nebenprodukte durch Filtration oder Zentrifugieren entfernt werden. Das nach der destillativen Entfernung des Lösungsmittels als Destillationsrückstand anfallende Rohprodukt kann gewünschtenfalls durch Umkristallisieren aus einem geeigneten inerten Lösungsmittel, beispielsweise Toluol oder vorzugsweise destillativ gereinigt werden.

Obwohl es sich bei den erfindungsgemäßen Diisocyanaten um thermostabile Substanzen handelt, kann es zweckmäßig sein, die Reindestillation der Diisocyanate ohne große Temperaturbelastung, beispielsweise mittels eines Dünnschichtverdampfers durchzuführen. Gewünschtenfalls können die erfindungsgemäßen Diisocyanate nach ihrer Reindarstellung auch durch Tempern bei Temperaturen von 160°C bis 250°C, vorzugsweise 180°C bis 230°C, von störenden Nebenprodukten, beispielsweise thermolabilen, chlorhaltigen Verbindungen befreit werden.

Bei der erfindungsgemäßen Verwendung der neuen Diisocyanate zur Herstellung von Polyurethankunststoffen, insbesondere von massiven oder zellförmigen Polyurethaneelastomeren werden die erfindungsgemäßen Diisocyanate anstelle der bislang für diesen Verwendungszweck eingesetzten Diisocyanate mit den bekannten Reaktionspartnern zur Umsetzung gebracht (vgl. diesbzüglich beispielsweise die bereits eingangs genannten Literaturstellen oder auch "Kunststoff-Handbuch", Band VII, "Polyurethane" von Vieweg und Höchtlen, Carl Hanser Verlag München (1966), insbesondere Seiten 206—297).

So erfolgt beispielsweise die Herstellung von Polyurethanelastomeren unter Verwendung des erfindungsgemäßen Diisocyanats durch dessen Umsetzung mit

a) Di- oder trifunktionellen Polyhydroxylverbindungen des Molekulargewichtsbereichs 400 bis 10.000, vorzugsweise 800 bis 3.000, vorzugsweise den entsprechenden Polyhydroxypolyestern oder Polyhydroxypolyethern,

b) Kettenverlängerungsmitteln des Molekulargewichtsbereichs 60 bis 399, d.h. mit im Sinne der Isocyanat-Additionsreaktion difunktionellen Verbindungen mit alkoholischen Hydroxylgruppen oder primären bzw. sekundären Aminogruppen, gegebenenfalls in Gegenwart von

c) weiteren aus der Chemie der Polyurethanelastomeren an sich bekannten Hilfs- und Zusatzmitteln.

Die Umsetzung kann nach dem bekannten Prepolymerverfahren (Umsetzung des Diisocyanats mit der Komponente a) unter Einhaltung eines Äquivalentverhältnisses von Isocyanatgruppen zu gegenüber Isocyanatgruppen reaktionsfähigen Gruppen von größer als 1,3:1 und anschließende Umsetzung des so erhaltenen NCO-Prepolymeren mit der Komponente b) oder aber auch einstufig durch Umsetzung des Diisocyanats mit einem Gemisch der Komponente a) und b) erfolgen. Bei beiden Varianten liegt das Äquivalentverhältnis von Isocyanatgruppen zur Gesamtmenge der gegenüber Isocyanatgruppen reaktionsfähigen Gruppen im allgemeinen bei 0,8:1 bis 1,3:1, vorzugsweise 0,95:1 bis 1,1:1. Die Temperaturen, bei welchen diese Umsetzungen durchgeführt werden, liegen im allgemeinen bei 60 bis 180°C, vorzugsweise bei 80 bis 150°C. Die Umsetzungen können in Anwesenheit oder auch in Abwesenheit von geeigneten inerten Lösungsmitteln erfolgen.

Bei den mit den erfindungsgemäßen Diisocyanaten hergestellten Polyurethankunststoffen, insbesondere Polyurethanelastomeren kann es sich sowohl um massive als auch um zellförmige Produkte handeln. Die Herstellung beider Typen von Polyurethanelastomeren erfolgt nach den bekannten Verfahren, wie sie beispielsweise in der zuletzt genannten Literaturstelle beschrieben sind. So erfolgt beispielsweise die Herstellung von zellförmigen Polyurethanelastomeren unter Verwendung bzw. Mitverwendung von Wasser als Kettenverlägerungsmittel.

Die mit den erfindungsgemäßen Diisocyanaten hergestellen Kunststoffe, insbesondere Elastomeren, die mit solchen erfindungsgemäßen Diisocyanaten hergestellt sind, besitzen hochwertige mechanische

und thermische Eigenschaften. Sie sind deshalb hervorragend geeignet für Feder- und Dämpfungselemente, Puffer, Radbeläge, Dichtungen, Schuhsohlen und ähnliche Einsatzgebiete, bei denen das Material extremen mechanischen und thermischen Beanspruchungen ausgesetzt ist.

Das folgende Beispiel soll die Erfindung näher erläutern. Prozentangaben beziehen sich auf Gewichtsprozente.

Beispiel 1

Herstellung von 1,4-Bis-(4-isocyanatophenoxy)-benzol

In einer 6 1-Laborphosgenierappatatur wurde eine Lösung von 350 g Phosgen in 3,8 l wasserfreiem Chlorbenzol vorgelegt und unter Rühren und Kühlung bei 0 bis 10°C mit 328 g 1,4-Bis-(4-aminophenoxy)-benzol (analytisch betimmte Reinheit: 97,1%) versetzt. Unter Einleiten von zusätzlichem Phosgen (ca. 50 g/h) wurde das Reaktionsgemisch im Verlauf von 2,5 Stunden zum Rückfluß erhitzt, wobei eine klare Lösung entstand. Nach weiteren 1,5 Stunden Phosgenieren am Rückfluß wurde das fertige Reaktionsgemisch destilativ vom überschüssigen und vom Lösungsmittel befreit. Das zurückbleibende Rohprodukt wurde durch Destillation im Vakuum gereinigt. Dabei destillierte 1,4-Bis-(4-isocyanatophenoxy)-benzol ohne Vorlauf bei 0,2 mbar und 210°C bis 215°C als farblose Flüssigkeit über, die rasch zu Kristallen vom Schmelzpunkt 115°C bis 116°C erstarrte.

Ausbeute: 475 g (94,8% der Theorie)

$C_{20}H_{12}N_2O_4$ (344,3)

NCO-Gehalt: ber. 24,4% gef. 24,3/24,5%

Destillationsrückstand: 33 g dunkel gefärbte Masse

Das Diisocyanat ließ sich rückstandsfrei redistillieren und ergab farblose Kristalle mit unverändertem Schmelzpunkt und einem Gehalt an hydrolysierbarem Chlor von 70 ppm.

Beispiel 2

Herstellung von Kunststoffen unter Verwendung von Diisocyanaten

Ein Polyester aus Adipinsäure und Ethylenglykol wurde im Vakuum eine Stunde bei 80 bis 100°C und 18 mbar entwässert. Von diesem Polyester (OH-Zahl: 56) wurden jeweils 200 g (0,1 mol) in einer Rührapparatur bei 70—80°C vorgelegt. Unter Rühren wurde das Polyester mit jeweils 0,18 mol eines der folgenden Diisocyanate in geschmolzener Form versetzt:

1,5-Diisocyanatonaphtalin (NDI, Vergleich)

1,4-Bis-(4-isocyanatophenoxy)benzol (DIP, erfindungsgemäß)

4,4'-Diisocyanato-diphenylmethan (MDI, Vergleich)

Anschließend wurden die Gemische 1 bis 2 Stunden unter Rühren bei 80°C gehalten und so zu den Prepolymeren umgesetzt. Danach wurden die Produkte 5 Minuten durch Anlegen von Vakuum entgast und unter Rühren mit jeweils 6,0 g (67 mmol) 1,4-Butandiol versetzt. Die Produktgemische wurden in mit Trennmittel versehene Formen gegossen und bei 120°C ausgeheizt und verfestigt. Nach der Entformung wurden die Formteile noch 4 Stunden bei 129°C nachgetempert und dann untersucht.

| Meßergebnisse: | NDI | DIP | MDI |
|---|---|---|---|
| Härte (Shore A) | 87 | 82 | 58 |
| Modul 100% (MPa) | 4,7 | 4,5 | 1,9 |
| Zugfestigkeit (MPa) | 29,8 | 27,8 | 18,2 |
| Stoßelastizität (%) | 45 | 52 | 32 |
| Bruchdehnung (%) | 750 | 650 | 850 |

Wie aus den Meßergebnissen zu ersehen ist, liefern die erfindungsgemäßen Diisocyanate Elastomere, die vergleichbar und zum Teil besser sind als mit 1,5-Diisocyanatonaphtalin hergestellte Elastomere.

**Patentansprüche**

1. 1,4-Bis-(4-isocyanatophenoxy)-benzole der allgemeinen Formel

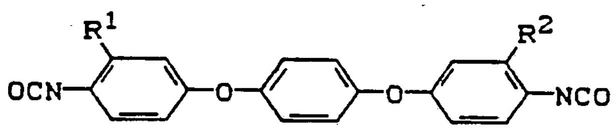

in der

$R^1$ und $R^2$ gleich oder verschieden sind und die Bedeutung eines Wasserstoffatoms oder einer Methylgruppe haben.

2. Verfahren zur Herstellung von 1,4-Bis-(4-isocyanatophenoxy)-benzolen der genannten Formel, dadurch gekennzeichnet, daß man 1,4-Bis-(4-aminophenoxy)-benzole der allgemeinen Formel

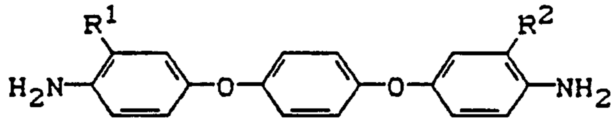

in der $R^1$ und $R^2$ die genannte Bedeutung haben, oder deren Addukte mit Chlorwasserstoff oder Kohlendioxid in an sich bekannter Weise mit Phosgen umsetzt.

3. Verwendung von 1,4-Bis-(4-isocyanatophenoxy)-benzolen der in Anspruch 1 genannten Formel als Aufbaukomponente bei der Herstellung von Polyurethankunststoffen nach dem Isocyanat-Polyadditionsverfahren.

**Revendications**

1. 1,4-bis-(4-isocyanatophénoxy)-benzènes de formule générale

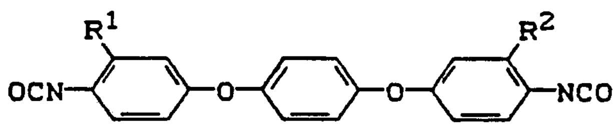

dans laquelle

$R^1$ et $R^2$ sont identiques ou différents et représentent un atome d'hydrogène ou un groupe méthyle.

2. Procédé de production de 1,4-bis-(4-isocyanatophénoxy)-benzènes de la formule mentionnée, caractérisé nen ce qu'on fait réagir avec le phosgène d'une manière connue des 1,4-bis-(4-aminophénoxy)-benzènes de formule générale

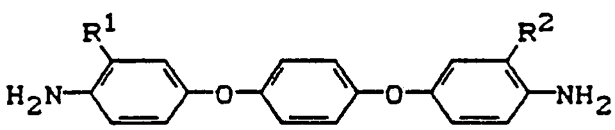

dans laquelle $R^1$ et $R^2$ ont la définition mentionée, ou leurs produits d'addition avec le chlorure d'hydrogène ou l'anhydride carbonique.

3. Utilisation de 1,4-bis-(4-isocyanatophénoxy)-benzènes de formule mentionnée dans la revendication 1 comme composant structural dans la production de matières plastiques du type polyuréthanne par le procédé de polyaddition d'isocyanate.

**Claims**

1. 1,4-Bis-(4-isocyanatophenoxy)-benzenes corresponding to the following general formula

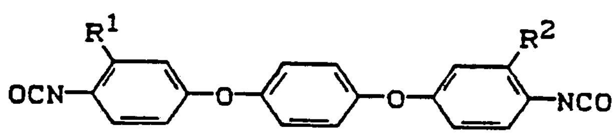

in which

$R^1$ and $R^2$ may be the same or different and represent a hydrogen atom or a methyl group.

2. A process for the production of 1,4-bis-(4-isocyanatophenoxy)-benzenes corresponding to the above formula, characterized in that 1,4-bis-(aminophenoxy)-benzene corresponding to the following general formula

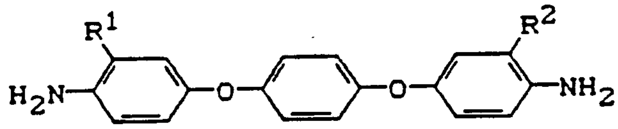

in which $R^1$ and $R^2$ are as defined above, or adducts thereof with hydrogen chloride or carbon dioxide are reacted with phosgene in known manner.

3. The use of 1,4-bis-(4-isocyanatophenoxy)-benzenes corresponding to the formula in claim 1 in the production of polyurethane plastics by the isocyanate polyaddition process.